# EUROPEAN PATENT APPLICATION

(11) **EP 4 019 042 A1**
(43) Date of publication of application: **29.06.2022**
(21) Application number: 20383155.7
(22) Date of filing: 23.12.2020
(51) Int. Cl.: A61K 39/12, A61K 39/215, A61K 49/00, C07K 4/02

(54) **METHOD FOR DETERMINING WHETHER AN IMMUNE RESPONSE HAS OCCURRED IN SUBJECTS WHO HAVE BEEN INFECTED WITH- OR VACCINATED AGAINST CORONAVIRUS**

(71) Applicant: De La Cuesta Roldán, Carlos, 28232 Las Rozas de Madrid (ES); Vidal, Nicolás Alejandro, 4200 Santiago del Estero (AR)
(72) Inventor: SERRANO, Julián Alberto, G4200 Santiago del Estero (AR)
(74) Representative: Hoffmann Eitle

(57) **Abstract**

The present invention refers to the medical field. Particularly, it refers to a method for determining whether an immune response has occurred in subjects who have been infected with- or vaccinated against coronavirus.

## Description

### FIELD OF THE INVENTION

The present invention refers to the medical field. Particularly, it refers to a method for determining whether an immune response has occurred in subjects who have been infected with- or vaccinated against coronavirus.

### PRIOR ART

A week after alerting the WHO of a cluster of pneumonia of unknown etiology in Wuhan, the Chinese authorities announced on 7 January 2020 that a novel Coronavirus was identified as the cause of these pneumonia. According to phylogenetic analysis, this novel severe acute respiratory syndrome Coronavirus 2 (SARS-CoV-2), previously named 2019-nCoV, belongs to the B lineage of Betacoronavirus genus and the Sarbecovirus subgenus and has more than 85% nucleotide sequence identity with a bat SARS-like CoV genome published previously. On January 30, WHO declared coronavirus disease-19 (COVID-19) outbreak a public health emergency of international concern and the disease has now spread worldwide.

The COVID-19, currently in pandemic status, caused by SARS-CoV-2, has spread rapidly around the world since the first cases were reported in late 2019. Currently, the number of people who have already overcome the infection is around 78 million worldwide and it is possible that a significant number of people who had the infection asymptomatically were not captured by the corresponding health systems.

One of the strategies to detect all those people who have developed immunity against the virus, after having overcome the infection, is the evaluation of serum antibodies levels against SARS-CoV-2 proteins, particularly protein S or Spike. Thus, tests that detect this antibody response (known as humoral immunity) have been rapidly developed. The information provided by the follow-up of people who have developed immunity helps to estimate the burden of disease, the dynamics of transmission, and the modelling of the epidemic.

However, based on the results reported in different studies, it has been possible to verify that the humoral response, both of IgM and IgG, shows a peak and subsequently declines, without remaining at the long term expected levels. Thus, following 4 to 10 weeks after the disappearance of symptoms, the levels of antibodies show a rapid reduction of the IgG values by half. This evidence raises serious concerns about the robustness and sustainability of the humoral immune response in the post-recovery period, which had been considered crucial for immunity strategy and vaccine development.

Additionally, some people were reported with PCR (polymerase chain reaction) positive for COVID-19 and undetectable levels of protective IgG antibodies, or neutralizing antibodies at very low titters.

On the other side, the emergency of developing vaccination strategies for a disease that is not fully understood has revealed the risk of vaccinating individuals with a high risk of atopy allergy. Huge numbers of people suffer from allergic responses, which can vary in significance from being mildly inconvenient to resulting in rapid death. Allergic responses due to type I hypersensitivity are often referred to as immediate hypersensitivity or atopic allergy and are mediated by the IgE class of antibody bound to tissue mast cells and to circulating blood basophils. Cross-linking of the IgE by the antigen (called allergen in this case) results in the release of a plethora of inflammatory mediators that cause the symptoms. If the allergen reaches the blood stream, the potential death risk increases. Although severe hypersensitivity events are rare, since many countries in the world now recommend and enforce mandatory vaccinations to improve COVID-19 vaccination coverage, the number of hypersensitivity reactions to these vaccines may show an increase. The confirmation of an IgE-mediated response implies recommending that the subject should not be vaccinated.

Thus, in one sense, there is an unmet medical need of finding cost-effective methods to ascertain whether subjects who have been infected with-, or have been vaccinated against Coronavirus, are eliciting an appropriate immune response, for instance a cellular or humoral immune response. On the other, no current methods can detect vulnerable subjects to developing allergic reactions to Coronavirus vaccines. These cost-effective methods could be used for detecting subjects who have already overcome the infection, for evaluating the efficacy of new vaccines, for establishing a priority order among the candidates to receive vaccination, for determining prognostic value of the cellular immune response during the infection, or for assessing possible severe type I hypersensitivity reaction to the vaccine before recommending whether a specific subject should be vaccinated.

The present invention is focused on solving the above cited problem, and a method for identifying immune response in subjects who have been infected with- or vaccinated against Coronavirus is herein described. Kindly note that we are not aware of skin tests for assessing immune response in the context of respiratory viral infections. Thus, the finding of reactive intradermal reaction (IDR) for the SARS-CoV-2 virus would be, to our knowledge, the first case that uses this technique to evaluate an immune response to a respiratory viral infection. Furthermore, our technique offers the advantage of also being able to detect individuals with type I hypersensitivity in whom the vaccine is potentially contraindicated.

### DESCRIPTION OF THE INVENTION

### Brief description of the invention

As explained above, the present invention is directed to a method (hereinafter method of the invention) for determining whether an immune response has occurred in subjects who have been infected with- or vaccinated against Coronavirus.

The method of the invention is a cost-effective procedure which could be used in different scenarios, comprising point-of-care testing. The information provided by the method of the invention regarding whether the subjects are eliciting an immune response against Coronavirus insults could be used for different purposes, for instance: for detecting subjects who have already overcome the infection, for evaluating the efficacy of new vaccines, for establishing a priority order among the candidates to receive vaccination, for determining prognostic value of the cellular immune response during the infection or for assessing possible severe type I hypersensitivity reaction to the vaccine before recommending whether a specific subject should be vaccinated.

So, the first embodiment of the present invention refers to Coronavirus-derived antigen/s for use in a method for determining whether an immune response has occurred in subjects who have been infected with- or vaccinated against Coronavirus which comprises: a) Determining the presence of a wheal or skin induration once the Coronavirus-derived antigen has been administered intradermally, and b) wherein the presence of a wheal or skin induration is an indication that the subjects have developed immune response against the Coronavirus infection. Otherwise, this embodiment also refers to a method for determining whether an immune response has occurred in subjects who have been infected with- or vaccinated against Coronavirus which comprises: a) Intradermal administration of Coronavirus-derived antigen/s, b) determining the presence of a wheal or skin induration once the Coronavirus-derived antigen has been administered intradermally, and c) wherein the presence of a wheal or skin induration is an indication that the subjects have developed immune response against the Coronavirus infection.

Thus, the present invention can be used for detecting any kind of immune response which may occur once the subjects have been infected with- or vaccinated against Coronavirus, for instance (non-exhaustive list): Humoral immune response mediated by antibody molecules, preferably IgE-mediated immune response, or cellular immune response mainly mediated by T cells (cell-mediated immunity).

In a preferred embodiment the present invention refers to Coronavirus-derived antigen/s for use in a method for determining whether an IgE-mediated immune response has occurred in subjects who have been infected with- or vaccinated against Coronavirus which comprises: a) Determining the presence of a wheal once the Coronavirus-derived antigen has been administered intradermally, and b) wherein the presence of a wheal between 10-30 minutes following the antigen administration is an indication that the subjects have developed IgE-mediated immune response against the Coronavirus-derived antigen/s. The observation of the wheal at 10-30 minutes after administration evaluates the possible occurrence of IgE-mediated hypersensitivity (also named immediate hypersensitivity, type I hypersensitivity or atopic allergy) to the antigen administered.

In a preferred embodiment the present invention refers to Coronavirus-derived antigen/s for use in a method for recommending whether a subject should be vaccinated against Coronavirus which comprises: a) Determining the presence of a wheal once the Coronavirus-derived antigen has been administered intradermally, and b) wherein the presence of a wheal between 10-30 minutes following the antigen administration is an indication that the subjects have developed IgE-mediated immune response against the Coronavirus-derived antigen/s and the vaccination of these subjects is potentially contraindicated. So, the present invention also refers to a method for the prophylactic treatment of subjects by means of the administration of a vaccine against Coronavirus which comprises a first or previous step of determining whether an IgE-mediated immune response has occurred according to the procedure described above. In this regard, although vaccination would be usually contraindicated when an IgE-mediated immune response has been identified, the subject could be even vaccinated as far as the vaccine is applied by an experienced staff and with specific precautions.

In a preferred embodiment the present invention refers to Coronavirus-derived antigen/s for use in a method for determining whether cell-mediated immune response has occurred in subjects who have been infected with- or vaccinated against Coronavirus which comprises: a) Determining the presence of a skin induration once the Coronavirus-derived antigen has been administered intradermally, and b) wherein the presence of a skin induration between 24-72 hours following the antigen administration is an indication that the subjects have developed cellular immune response against the Coronavirus infection.

In a preferred embodiment the present invention refers to Coronavirus-derived antigen/s for use in a method for determining whether cell-mediated immune response has occurred in subjects who have been infected with- or vaccinated against Coronavirus which comprises: a) Determining the presence of a skin induration once the Coronavirus-derived antigen has been administered intradermally, and b) wherein the presence of a skin induration with a diameter of its major axis, excluding the erythema usually formed around the skin induration, longer than a pre-established threshold value determined in control subjects who have not developed cellular immune response, preferably a diameter of the major axis of at least 4.00 mm, is an indication that the subjects have developed cellular immune response against the Coronavirus infection.

In a preferred embodiment of the present invention, the Coronavirus infection is caused by SARS-CoV-2 and the antigen is a molecule derived thereof which is able to elicit an immune response in the host, comprising (non-exhaustive list): Structural proteins selected from Spike (S), Nucleocapsid (N), Membrane (M) or Envelope (E) protein; inactivated or attenuated viral particles; isolated genetic material or virus-like particles.

In a preferred embodiment of the present invention, the antigen is included in a pharmaceutical composition which comprises an effective amount of the Coronavirus-derived antigen and, optionally, comprises pharmaceutically acceptable excipients and/or carriers. In a preferred embodiment, the doses of Coronavirus derived antigen which is applied to the patient is between 0.050 µg/ 0.1 mL and 0.500 µg/0.1 mL.

In a preferred embodiment, the Coronavirus-derived antigen is administered by any means able to reach the intradermal space, for example by means of an intradermal injection or a patch test.

In a preferred embodiment the present invention comprises: a) Entering into a computer or device the diameter of the major axis of the wheal or the skin induration, excluding the erythema usually formed around the wheal or skin induration, once the Coronavirus-derived antigen has been administered intradermally; preferably by means of an image taken from the skin, and b) providing a result by the computer or device system regarding the development of immune response based on the information entered according to the step a) and a pre-established threshold value previously stored in the computer, wherein this result is communicated by the computer system to the subject and/or to the authorized health personnel and/or the health system.

The second embodiment of the present invention refers to a method for obtaining information regarding whether an immune response has occurred in subjects who have been infected with- or vaccinated against Coronavirus which comprise performing the steps disclosed above.

The third embodiment of the present invention refers to a data processing apparatus, device or system comprising means for carrying out the method of the invention.

The fourth embodiment of the present invention refers to a computer program product configured for carrying out the method of the invention.

The fifth embodiment of the invention refers to a kit which comprises: a) A medical device, for instance a syringe, for administering intradermally a Coronavirus-derived antigen, and b) instructions for determining the presence of a wheal or skin induration.

In a preferred embodiment the present invention refers to a kit which comprises a) A medical device for administering intradermally a Coronavirus-derived antigen, and b) instructions for determining the presence of a wheal between 10-30 minutes following the antigen administration. The present invention also refers to the use of this kit for determining whether an IgE-mediated immune response has occurred in subjects who have been infected with- or vaccinated against Coronavirus, or for recommending whether a subject should be vaccinated against Coronavirus.

In a preferred embodiment the present invention refers to a kit which comprises a) A medical device for administering intradermally a Coronavirus-derived antigen, and b) instructions for determining the diameter of the major axis of the skin induration formed between 24-72 hours following the antigen administration. The present invention also refers to the use of this kit for determining whether cell-mediated immune response has occurred in subjects who have been infected with- or vaccinated against Coronavirus.

For the purpose of interpreting the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of' means including, and it is limited to, whatever follows the phrase "consisting of'. Thus, the phrase "consisting of' indicates that the listed elements are required or mandatory, and that no other elements may be present.
- By "effective dose or amount" is intended an amount that, when administered as described herein, brings about a measurable immune response in the host. The exact amount required will vary from subject to subject, depending on the age, and general condition of the subject, the severity with which the disease has been/is progressed, mode of administration, and the like. An appropriate "effective" amount in any individual case may be determined by one of ordinary skill in the art using routine experimentation, based upon the information provided herein.
- A reference control level is mentioned herein, when referring to the skin induration diameter. The subject is likely to elicit a cellular immune response with a given sensitivity and specificity if the skin induration diameter in its major axis (excluding the erythema) is above said reference control level. A reference value can be a "threshold value" or a "cut-off" value. Typically, a "threshold value" or "cut-off value" can be determined experimentally, empirically, or theoretically. According to the present invention, the "threshold" value refers the skin induration diameter identified in subjects which has not elicit a cellular immune response.
- The expression "determining the presence of a wheal", particularly refers to the method for determining whether an IgE-mediated immune response has occurred. This is a qualitative method and is based on determining whether a wheal is present or absent within 10-30 minutes following the antigen administration. So, in this context, the presence of the wheal within said period is enough to conclude that an IgE-mediated immune response has been generated.
- By "major axis of the wheal or skin induration" it is understood the greatest distance between two opposite points on the wheal or skin induration which is formed when the coronavirus-derived antigen is administered to the subject.

### Brief description of the figures

**Figure 1**: Serum IgG levels anti-protein S from SARS-CoV-2 among COVID-19 and control cases. *****p* < .0001, unpaired Student's *t*-test.
**Figure 2****.** IDR measurement after 24h and 48h of an i.d. injection of S protein at different doses. The induration induced by the IDR is reported as the diameter of its major axis, without the surrounding erythema. *****p* < .0001, unpaired Student's *t*-test.
**Figure 3****.** Intradermal reaction specific to SARS-CoV-2 virus S protein in 9 convalescent subjects infected with SARS-CoV-2. Of the 9 subjects studied, 7 had mild symptoms while 2 showed severe symptoms, and only 4 were evaluated at two time points. The magnitude of *in vivo* responses for each individual was evaluated 48 (a) or 24 (b) hours following the i.d. injection. Each coloured segment represents a different dose of protein i.d. injected in 0.1 mL of vehicle.

### Detailed description of the invention

The present invention is illustrated by means of the Examples set below, without the intention of limiting its scope of protection.

### Example 1. Materials and Methods

Humans: Volunteers were recruited based on their clinical history of SARS-CoV-2 infection (diagnosed by positive PCR for the SARS-CoV-2 virus in nasopharyngeal swab). The studies on subjects who recovered from COVID-19 (n = 9) were tested after the symptom's onset. Healthy subjects without a clinical history of COVID-19 (n = 6) were simultaneously recruited, between October and December 2020. All healthy donor samples tested negative for IgG antibodies to S protein.

Inclusion criteria: Individuals aged 20 to 76 years, of both sexes.

Exclusion criteria: Individuals who suffer infectious diseases, acute or chronic inflammatory diseases, treatment with immunomodulatory drugs (systemic corticosteroids), cancer, kidney failure, heart failure, immunodeficiencies or autoimmune diseases.

Ethical and legal aspects: Volunteers were informed of the purpose and methods of the study in order to obtain their written consent, developed in compliance with the Declaration of Helsinki and the Good Clinical Practice Guidelines.

Reagents and materials: Protein S of the SARS-CoV-2 virus were used, dissolved in a vehicle solution composed of phosphate buffer saline (PBS, pH = 7.4) and different conservatives. The vehicle solution was used as a negative control of the reaction. Sterile 1mL syringes and hypodermic needles were used for intradermal injection (i.d.), as described below.

Computer application: Applications for iPhone and Android cell phones to capture images and an artificial intelligence to analyse images were developed.

Humoral response: On day 1, peripheral blood was obtained by venipuncture previous to the procedure (see below). The samples were centrifuged for 5 minutes at 2000 r.p.m. and the serum separated in order to determine the levels of IgG anti-protein S by chemiluminescence as a parameter for humoral response.

### Example 2. Procedure

Intradermal reaction (IDR): On day 1, 0.1 mL of the solution containing protein S from SARS-CoV-2 were i.d. injected on the anterior surface of the forearm, previously disinfected with a suitable germicide, which were allowed to dry before injection. The presence of a wheal or skin induration in the injection area was evaluated 10-30 minutes and 24 hours and 48 hours after the injection (days 2 and 3, respectively). At 10-30 minutes following administration, the presence or absence of a wheal was reported. At 24 hours and 48 hours following administration, the diameter of the major axis of the skin induration (excluding the erythematous area) was measured in millimetres (mm) with a graduated ruler in a double-blind manner. If there were, the presence and size of lesions or edema were also recorded.

Dose-response curve: 3 injections were applied in each subject with different doses of protein, according to the following schedule:
A: 0 µg/0.1 mL
B: 0.145 µg/0.1 mL
C: 0.28 µg/0.1 mL

### Statistical Analysis

For comparison, two tailed Student's t-tests were applied unless otherwise specified. Statistical tests were performed using GraphPad.7 for Windows.

### Example 3. Results

### Clinical Characteristics

**Table 1** shows the clinical characteristics of the 9 confirmed cases (56% male, 44% female), who were diagnosed by the Ministry of Public Health of Santiago del Estero, Argentina, between 3 August 2020 and 17 November 2020, with mean 111 (± 28) days after onset of symptom, and 6 control cases (50% male, 50% female). In COVID-19 cases, the average age was 41.9 (± 8.7) years and no subjects older than 60, while in control cases the average age was 49.2 (± 13.1) years and 1 subject older than 60. All subjects were residents of Santiago del Estero city, Argentina, or surrounding areas. Among COVID-19 cases, 1 (11%) had underlying hypothyroidism and overweight, and 2 (22%) were smokers, while among controls, only 1 (17%) smoker was reported.

**Table 1**

| **Subject ID** | **Group** | **Age** | **Gender** | **Comorbidities** | **COVID-19 severity** | **Days post-PCR** |
|---|---|---|---|---|---|---|
| Con1 | Control | 46 | F | - | - | - |
| Con2 | Control | 76 | M | - | - | - |
| Con3 | Control | 52 | M | - | - | - |
| Con4 | Control | 42 | M | Smoker | - | - |
| Con5 | Control | 34 | F | - | - | - |
| Con6 | Control | 45 | F | - | - | - |
| Cov1 | COVID-19 | 49 | F | Hypothyroidism Overweight | Mild | 96 |
| Cov2 | COVID-19 | 41 | M | - | Mild | 23 |
| Cov3 | COVID-19 | 34 | F | - | Asymptomatic | 127 |
| Cov4 | COVID-19 | 44 | F | - | Moderate | 129 |
| Cov5 | COVID-19 | 47 | M | Smoker | Mild | 59 |
| Cov6 | COVID-19 | 44 | F | - | Moderate | 128 |
| Cov7 | COVID-19 | 47 | M | Smoker | Mild | 128 |
| Cov8 | COVID-19 | 21 | M | - | Mild | 128 |
| Cov9 | COVID-19 | 50 | M | - | Mild | 82 |

| | | | | | | |
|---|---|---|---|---|---|---|
| *Clinical characteristics of the included subjects* | | | | | | |

### IgG anti-spike levels in COVID-19 cases

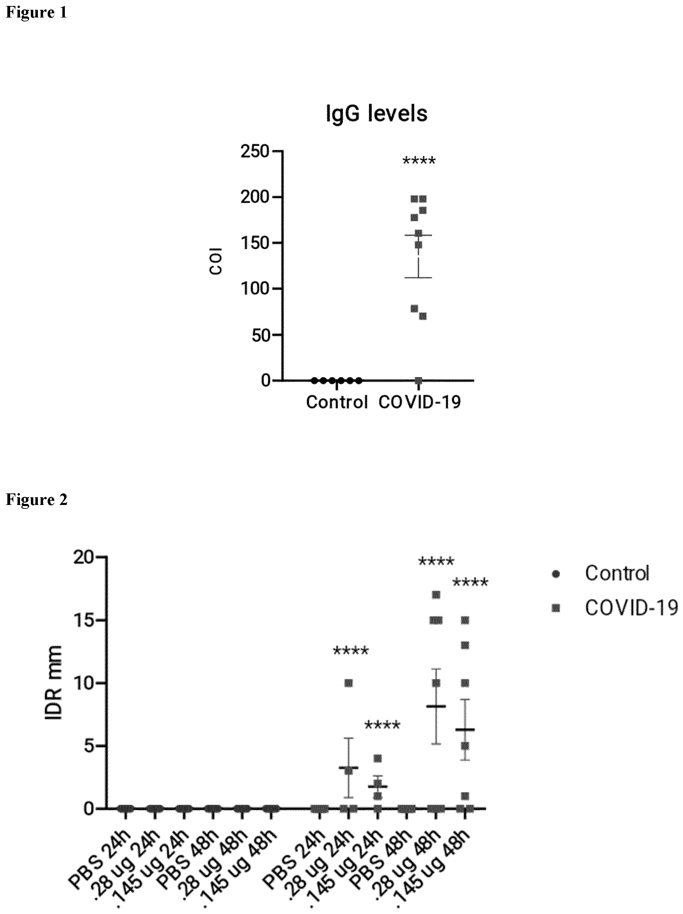

IgG anti-spike levels detected by chemiluminescence in serum samples obtained from COVID-19 and control cases at different periods after disease onset are shown in **Table 2.** Results are semi quantitative and are expressed as qualitative statements (reactive/non-reactive). A Cut-off-Index (COI) ≥ 1 is considered reactive. The average IgG levels was higher in COVID-19 group (.1 ± 0.00 COI) than in their control counterparts (135.2 ± 23.2 COI, **Figure 1**). Only 1 (11%) COVID-19 case did not show detectable IgG levels, as were also observed for all the control cases.

**Table 2**

| Subj ect ID | IDR 10-30 min (mm) | | | IDR 24h (mm) | | | IDR 48h (mm) | | | IgG levels (COI) | COVID-19 severity | Days post-PCR |
|---|---|---|---|---|---|---|---|---|---|---|---|---|
| | 0 µg | .145 µg | .28 µg | 0 µg | .145 µg | .28 µg | 0 µg | .145 µg | .28 µg | | | |
| Cov2 | 0 | 0 | 0 | 0 | 0 | 0 | 0 | 0 | 0 | 0.2 | M | 23 |
| Cov5 | 0 | 0 | 0 | Nd | Nd | Nd | 0 | 10 | 0 | 160.5 | M | 59 |
| Cov9 | 0 | 0 | 0 | Nd | Nd | Nd | 0 | 20 | 0 | 198 | M | 82 |
| Cov1 | 0 | 0 | 0 | 0 | 1 | 10 | 0 | 1 | 15 | 185.6 | M | 96 |
| Cov3 | 0 | 0 | 0 | 0 | 2 | 3 | 0 | 0 | 0 | 70.6 | A | 127 |
| Cov6 | 0 | 0 | 0 | Nd | Nd | Nd | 0 | 13 | 17 | 177.7 | Mo | 128 |
| Cov7 | 0 | 0 | 0 | Nd | Nd | Nd | 0 | 15 | 15 | 147.9 | M | 128 |
| Cov8 | 0 | 0 | 0 | Nd | Nd | Nd | 0 | 0 | 10 | 78.7 | M | 128 |
| Cov4 | 0 | 0 | 0 | 0 | 4 | 0 | 0 | 7 | 6 | 198 | Mo | 129 |
| Con1 | 0 | 0 | 0 | 0 | 0 | 0 | 0 | 0 | 0 | 0.1 | - | - |
| Con2 | 0 | 0 | 0 | 0 | 0 | 0 | 0 | 0 | 0 | 0.1 | - | - |
| Con3 | 0 | 0 | 0 | 0 | 0 | 0 | 0 | 0 | 0 | 0.1 | - | - |
| Con4 | 0 | 0 | 0 | 0 | 0 | 0 | 0 | 0 | 0 | 0.1 | - | - |
| Con5 | 0 | 0 | 0 | 0 | 0 | 0 | 0 | 0 | 0 | 0.1 | - | - |
| Con6 | 0 | 0 | 0 | 0 | 0 | 0 | 0 | 0 | 0 | 0.1 | - | - |

| | | | | | | | | | | | | |
|---|---|---|---|---|---|---|---|---|---|---|---|---|
| *IDR measures and serum IgG levels against Spike protein of SARS-CoV-2. The IDR values are shown in millimetres (mm) following 24h or 48h of i. d. injection of different doses of Spike protein. The days before symptoms onset and the severity of the disease are also shown. Cov, COVID-19 case; Con, Control case; Nd, Not determined; M, Mild; A, Asymptomatic; Mo, Moderate.* | | | | | | | | | | | | |

### Intradermal reaction (IDR)

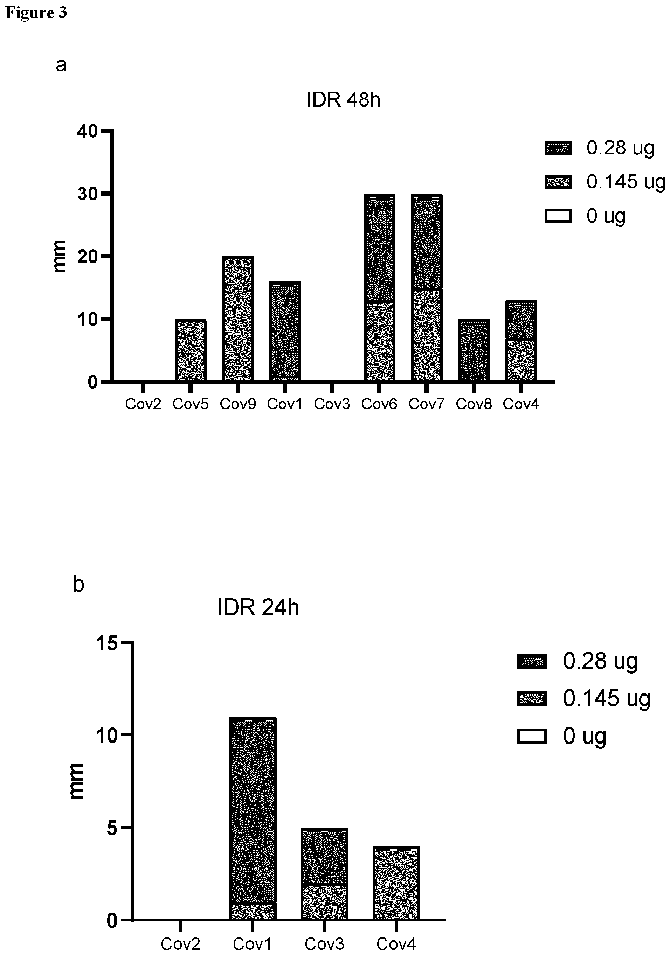

Between 10-30 minutes following injection, no wheals were observed in any case. Mean IDR size at 48h with 0.145 µg and 0.28 µg of S protein were 7.33 mm and 7.00 mm, respectively, referred as the diameter of the major axis of the skin induration, without erythema. Only 4 COVID-19 cases were also measured at 24h following injection, and the mean IDR size with 0.145 µg and 0.28 µg of S protein were 7.33 mm and 7.00 mm, respectively (**Figure 2**)**.** No differences were observed among COVID-19 and control group about vehicle (0 µg of protein) injection (**Table 2** and **Figure 3**). No lesions, edema, or unwanted effects were observed in any case.

## Claims

1. Coronavirus-derived antigen for use in a method for determining whether an immune response has occurred in subjects who have been infected with- or vaccinated against Coronavirus which comprises:
a. Determining the presence of a wheal or skin induration once the Coronavirus-derived antigen has been administered intradermally,
b. Wherein the presence of a wheal or skin induration is an indication that the subjects have developed immune response against the Coronavirus infection.

2. Coronavirus-derived antigen for use, according to claim 1, in a method for determining whether an IgE-mediated immune response has occurred in subjects who have been infected with- or vaccinated against Coronavirus which comprises:
a. Determining the presence of a wheal once the Coronavirus-derived antigen has been administered intradermally,
b. Wherein the presence of a wheal between 10-30 minutes following the antigen administration is an indication that the subjects have developed IgE-mediated immune response against the Coronavirus infection.

3. Coronavirus-derived antigen for use, according to claim 2, for recommending whether a subject should be vaccinated against Coronavirus which comprises:
a. Determining the presence of a wheal once the Coronavirus-derived antigen has been administered intradermally,
b. Wherein the presence of a wheal between 10-30 minutes following the antigen administration is an indication that the subjects have developed IgE-mediated immune response against the Coronavirus infection and the vaccination of these subjects is potentially contraindicated.

4. Coronavirus-derived antigen for use, according to claim 1, in a method for determining whether cell-mediated immune response has occurred in subjects who have been infected with- or vaccinated against Coronavirus which comprises:
a. Determining the presence of a skin induration once the Coronavirus-derived antigen has been administered intradermally,
b. Wherein the presence of a skin induration between 24-72 hours following the antigen administration is an indication that the subjects have developed cell-mediated immune response against the Coronavirus infection.

5. Coronavirus-derived antigen for use, according to claim 4, wherein the presence of a skin induration with a diameter of its major axis, excluding the erythema usually formed around the induration, longer than a pre-established threshold value determined in control subjects who have not developed cellular immune response, preferably a diameter of the major axis of a least 4.0 mm, is an indication that the subjects have developed cell-mediated immune response against the Coronavirus infection.

6. Coronavirus-derived antigen for use, according to any of the previous claims, wherein the Coronavirus infection is caused by SARS-CoV-2 and the antigen is a molecule derived thereof which is able to elicit an immune response in the host, comprising: Structural proteins selected from Spike (S), Nucleocapsid (N), Membrane (M) or Envelope (E) protein; inactivated or attenuated viral particles; isolated genetic material or virus-like particles.

7. Coronavirus-derived antigen for use, according to any of the previous claims, wherein the antigen is included in a pharmaceutical composition which optionally comprises pharmaceutically acceptable excipients and/or carriers.

8. Coronavirus-derived antigen for use, according to any of the previous claims, which comprises:
a. Entering into a computer the diameter of the major axis of the wheal or the skin induration, excluding the erythema usually formed around the wheal or the skin induration, once the Coronavirus-derived antigen has been administered intradermally; preferably by means of an image taken from the skin.
b. Providing a result by the computer system regarding the development of immune response based on the information entered according to the step a) and a pre-established threshold value already stored in the computer, wherein this result is communicated by the computer system to the subject and/or to the authorized health personnel and/or the health system.

9. A data processing apparatus, device or system comprising means for carrying out the method of claim 8.

10. Computer program product configured for carrying out the method of claim 8.

11. A kit which comprises:
a. A medical device for administering intradermally a Coronavirus-derived antigen, and
b. Instructions for determining the presence of a wheal or skin induration.

12. A kit, according to claim 11, which comprises:
a. A medical device for administering intradermally a Coronavirus-derived antigen, and
b. Instructions for determining the presence of a wheal between 10-30 minutes following the antigen administration.

13. A kit, according to claim 11, which comprises:
a. A medical device for administering intradermally a Coronavirus-derived antigen, and
b. Instructions for determining the diameter of the major axis of the skin induration formed between 24-72 hours following the antigen administration.

14. Use of the kit according to claim 12 for determining whether an IgE-mediated immune response has occurred in subjects who have been infected with- or vaccinated against Coronavirus, or for recommending whether a subject should be vaccinated against Coronavirus.

15. Use of the kit according to claim 13 for determining whether cell-mediated immune response has occurred in subjects who have been infected with- or vaccinated against Coronavirus.
